# EUROPEAN PATENT SPECIFICATION

(11) **EP 1 669 440 B1**
(45) Date of publication and mention of the grant of the patent: **02.12.2009**
(21) Application number: 04723331.7
(22) Date of filing: 25.03.2004
(51) Int. Cl.: C12N 5/00

(54) **COMPOSITION FOR COATING SUPPORT FOR PREPARATION OF CELL SHEET, SUPPORT FOR PREPARATION OF CELL SHEET AND PROCESS FOR PRODUCING CELL SHEET**
ZUSAMMENSETZUNG FÜR DIE BESCHICHTUNG EINES TRÄGERS ZUR HERSTELLUNG EINER ZELLAGE, TRÄGER ZUR HERSTELLUNG EINER ZELLAGE UND VERFAHREN ZUR HERSTELLUNG EINER ZELLAGE
COMPOSITION DE REVETEMENT DE SUPPORT POUR LA PREPARATION DE FEUILLE CELLULAIRE, SUPPORT POUR LA PREPARATION DE FEUILLE CELLULAIRE ET PROCEDE DE PRODUCTION DE FEUILLE CELLULAIRE

(30) Priority: 19.09.2003 JP 2003328340
(43) Date of publication of application: 14.06.2006
(73) Proprietor: Keio University, Tokyo 108-8345 (JP)
(72) Inventor: FUKUDA, Keiichi, c/o Keio University Sch. Medicine, Tokyo 160-8582 (JP); ITABASHI, Yuji, c/o Keio University Sch. Medicine, Tokyo 160-8582 (JP); TSUBOTA, Kazuo, Funabashi-shi, Chiba 273-0031 (JP)
(74) Representative: Woods, Geoffrey Corlett
(86) International application number: PCT/JP2004/004161
(87) International publication number: WO 2005/028638

(56) References cited:
- WO-A1-00/25838
- WO-A1-01/68799
- JP-A- 04 045 785
- JP-A- 2003 038 170
- SHIMIZU T. ET AL: 'Fabrication of pulsatile cardiac tissue grafts using a novel 3-dimensional cell sheet manipulation technique and temperature-responsive cell culture surfaces' CIRCULATION RESEARCH vol. 90, no. 3, 2002, pages E40 - E48, XP002285777

## Description

### Field of the Invention

The present invention relates to a composition for use in the coating of a substrate for cell sheet preparation, a substrate for cell sheet preparation, and a method for manufacturing a cell sheet.

### Background Art

In the treatment of a severely damaged heart, cell transplantation utilizing a variety of stem cells has been attempted as an alternative therapy to heart transplantation which has been suffering from shortage of donors. Recently, based on such cell transplantation techniques, tissue transplantation techniques have been increasingly developed in which myocardial tissues are constructed three-dimensionally in vitro and then transplanted into a body. For example, various types of cell sheets have been successfully manufactured by using temperature-responsive culture dishes which are prepared by coating poly(N-isopropylacrylamide) (abbreviated to "PIAAm") on the surfaces of commercial polystyrene culture dishes with electron beams. In particular, as for myocardial cells, it has already been reported that myocardial tissue masses available as transplants can be developed by overlaying the thus prepared multiple myocardial cell sheets (Japanese Patent Application Laid-open No. 2003-38170, WO 01/068799 pamphlet, Simizu et al. : Fabrication of pulsatile cardiac tissue grafts using a novel 3-dimensional cell sheet manipulation technique and temperature-responsive cell culture surface : Circ Res. 2002; 90:e40-e48). The thus prepared myocardial tissue mass is found to exhibit electrical activities similar to those of normal myocardial tissues in vitro and in vivo.

With respect to primary cultures of different tissues, particularly of myocardial cells, there is some difference in procedure among facilities. The method for manufacture of cell sheets using the above described temperature-responsive culture dishes may be effective for manufacturing cell sheets with a relatively consistent efficiency if the primary culture is performed by stringent procedures chosen for the best match to such specialized culture dishes. In this method, however, it is difficult to form sheets from cells if the procedures conventionally employed in each facility are applied without any modification.

Accordingly, an object of the present invention is to provide a method for manufacturing a cell sheet by a simple manipulation using a substrate coated with a commercial, commonly available material of which the safety has been confirmed.

Another object of the present invention is to provide a composition for use in the coating of a surface of a substrate for cell sheet preparation.

Still another object of the present invention is to provide a substrate suitable for cell sheet manufacture.

### Disclosure of the Invention

The present inventors have found that, when cells are cultured for several days on a culture dish which was previously given a light coating of fibrin glue (which is degradable by most cells), the fibrin between the cells and the culture dish disappears and, as a result, the cells are suspended over the culture dish while binding to one another in a sheet-like form. Then, the inventors have established a procedure for manufacturing cell sheets with high probability by detaching and harvesting the cell sheet intact with a scraper. These findings lead to the completion of the present invention.

The subjects of the present invention are as follows.
(1) A composition for use in coating with fibrin a surface of a substrate for cell sheet preparation, the composition comprising fibrinogen and thrombin.
(2) A substrate for cell sheet preparation, a surface of which is coated with fibrin.
(3) A method for manufacturing a cell sheet, comprising culturing cells on a fibrin-coated surface of a substrate until the cells reach confluency; continuing the cultivation of the cells for a sufficient time period to cause the degradation of fibrin at the bottom of the cells; and detaching the cultured cells from the substrate surface in a sheet-like form to give a cell sheet.
(4) The method according to item (3), further comprising overlaying the detached sheet-like cultured cells.
(5) The method according to item (3) or (4), wherein the cell sheet is used in the field of regenerative medicine or in a biological activity test of an agent.

The present invention enables cell sheets to be manufactured from a variety of cell types by employing the same procedures as those employed in various facilities without any modification, and the success rate of their manufacture is consistent.

The present invention also enables a large quantity of cell sheets to be manufactured quickly using commercial fibrin glues without the need to use expensive specialized PIAAm-coated culture dishes.

This specification includes part or all of the contents as disclosed in the specification and/or drawings of Japanese Patent Application No. 2003-328340 based on which the present application claims priority.

### Brief Description of Drawings

Fig. 1 shows the generation process of a rat myocardial cell sheet.
Fig. 2 shows the generation process of a C2C12 cell sheet.
Fig. 3 shows the generation process of a mature skeletal muscle cell sheet.
Fig. 4 shows electrocardiogram data of a myocardial cell sheet.
Fig. 5 shows a rat myocardial cell sheet transplanted onto the skin.
Fig. 6 shows the result of the immunostaining of a rat myocardial cell sheet with actinin and connexin 43 two days after establishing the sheet in vitro.
Fig. 7 shows the result of the HE staining of a rat myocardial cell sheet seven days after transplantation onto a nude rat subcutaneous tissue.
Fig. 8 shows the result of the immunostaining of a rat myocardial cell sheet with actinin and connexin 43 seven days after transplantation onto a nude rat subcutaneous tissue.
Fig. 9 shows a representative scheme of the mechanism and manipulation of a myocardial cell sheet using a polymerized fibrin-coated dish. The manipulation was performed in the order: A→B→C→D→E→F.
   Primary cultured neonate rat myocardial cells were spread onto a fibrin polymer-coated dish (A, B, G). On day 4, the fibrin polymer was degraded by various proteases secreted from the myocardial cells (C). The cells were gently raked from the edge toward the center of the dish so as not to tear the generated myocardial cell sheet (hereinafter, sometimes abbreviated as "MCS") with a cell scraper (D, E), thereby obtaining a shrunken MCS (H). A few drops of a culture medium were applied onto the shrunken sheet, whereupon it was unfolded or smoothed out (E, I). The edges of the flattened MCS were trimmed in a square shape by using a blade. In some experiments, two MCSs were overlaid on each other by the margin of 2-mm width and then co-cultured on a laminin-coated culture dish (F, J).

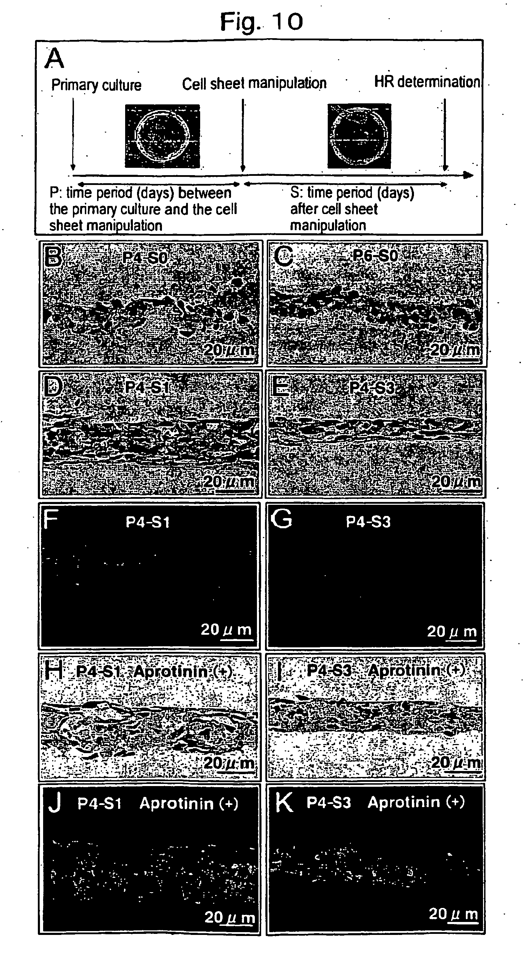

Fig. 10 shows the results of the histological analysis of a MCS.
   (A) The protocol for preparation and histological analysis of a myocardial cell sheet. (B-E, H, I) H&E staining of a MCS. (F, G, J, K) Immunofluorescent staining of a MCS. Red: F-actin-stained cell nuclei; Green: fibrin-stained cell nuclei; Blue: TOTO-3-stained cell nuclei. The protocol and scale bars are indicated in the figure inset.

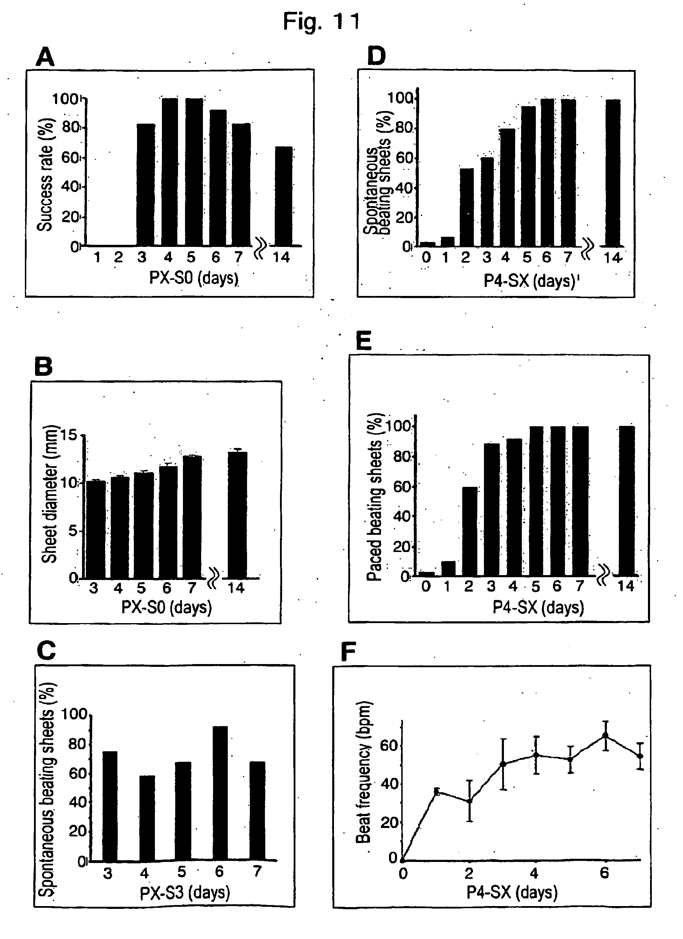

Fig. 11 shows the characteristic properties of MCSs.
   (A) Success rate for obtaining MCSs in the PX-S0 day samples. (B) Diameter of MCSs in PX-S0 samples. (C) Percentages of spontaneous beating in MCSs prepared using the PX-S3 day samples. (D) Percentage of spontaneously beating sheets from the P4-SX day samples. (E) Percentages of myocardial cell sheets that captured artificial pacing in the P4-SX day samples. (F) The beat frequency in the P4-SX day samples.

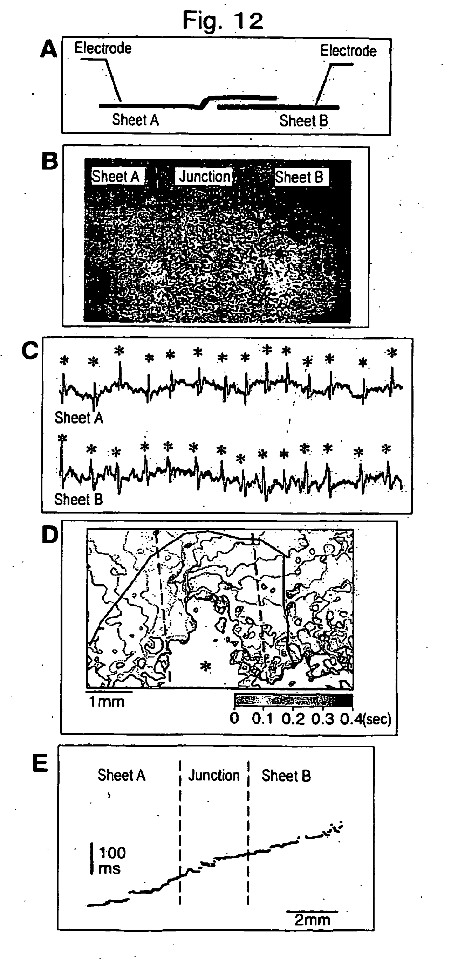

Fig. 12 shows the electrocardiogram (ECG) recorded using a pair of contact bipolar electrodes and the propagation of action potential recorded by optical mapping for analysis of electrical activities in two overlaid MCSs.
   (A) A schematic illustration of the two overlaid MCSs and the positions of the contact bipolar electrodes. (B) A microscopic image of the two overlaid MCSs. (C) Extracellular electrical potentials obtained from the two MCSs which showed synchronization in spontaneous beating. (D) A contour map of propagation of action potential as observed by optical mapping. The interval between each isochronal line was 35 ms. The action potential originated from the left lower side of sheet A, went around the lower half of the junction which was an electrically unexcitable area and propagated to sheet B via the upper localized half area of the junction. (E) Action potential as seen to propagate by tracing the excitation wave front (along the black curved line with arrowed head in D).

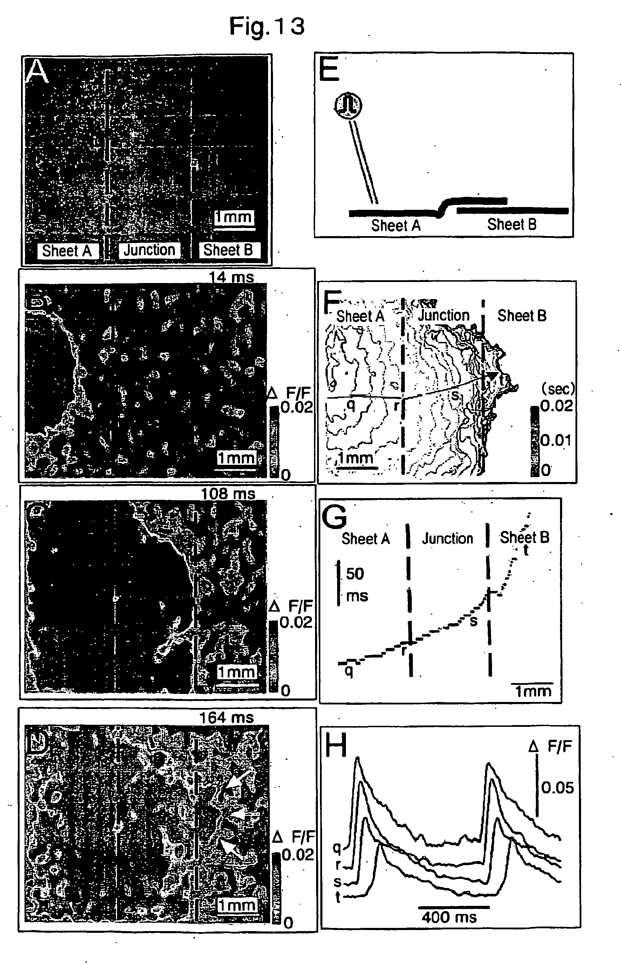

Fig. 13 is an optical mapping showing the action potential propagation of partially overlaid two MCSs one day after the co-culture of the MCSs was started.
   Representative data on day 1. (A) A photographic image observed under a phase contrast microscope. (B-D) Optical images showing active potential obtained at 14, 84, and 150 ms after pacing at the left margin of sheet A, respectively. Note that localized capture can be observed in sheet B (see the arrow). (E) A cross-sectional schematic image of the MCSs and the site of pacing. (F) An action potential map. The interval between each isochronal line is denoted 7 ms. There was crowding of the isochronal lines at the left margin of the junction, suggesting that conduction delay started to occur around this site. Action potential propagation was blocked at the end of the junction. (G) Impulse propagation sequence along the excitation wave front (see the red curved line with an arrow in F). (H) The action potential traces at arbitrary points along the excitation wave front were superposed. The characters correspond to the positions of the myocyte from which the action potential was recorded.

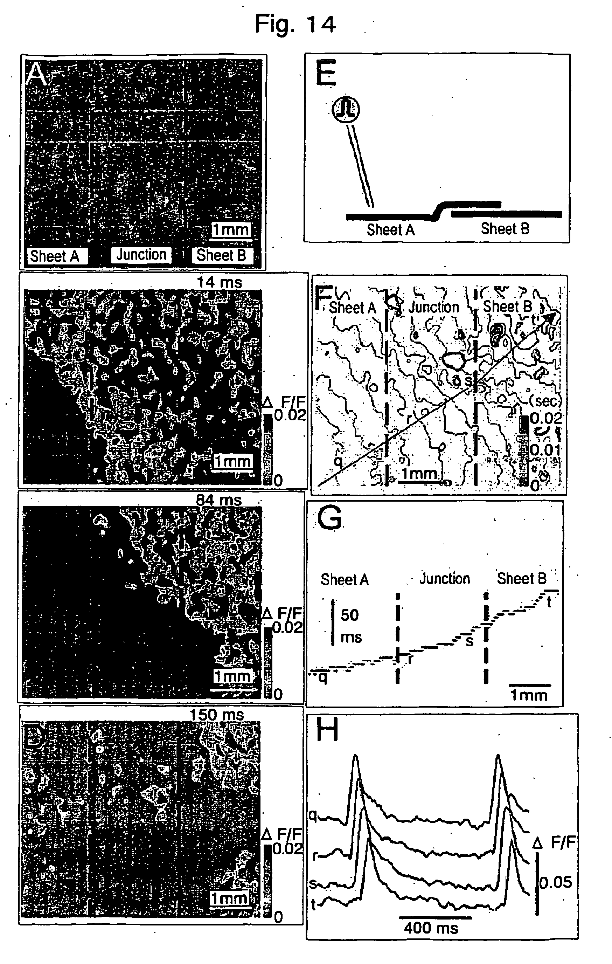

Fig. 14 is an optical mapping showing the action potential propagation and electrical connection of overlaid MCSs on day 3.
   Representative data on day 3. (A) A photographic image observed under a phase contrast microscope. (B-D) Optical mapping images showing active potential obtained at 14, 84, and 150 ms after pacing at the left margin of sheet A, respectively. (E) A cross-sectional schematic image of the MCSs and the site of pacing. (F) Calculated activation map (the size was the same as that in Fig. 13F), suggesting that the propagation of action potential between the two MCSs was quite smooth without any conduction delay. (G) The propagation sequence of the excitation wave front suggested the formation of tight electrical communication between the two MCSs without delay in the travel of excitation wave. (H) The action potential traces at arbitrary points along the excitation wave front were superposed.

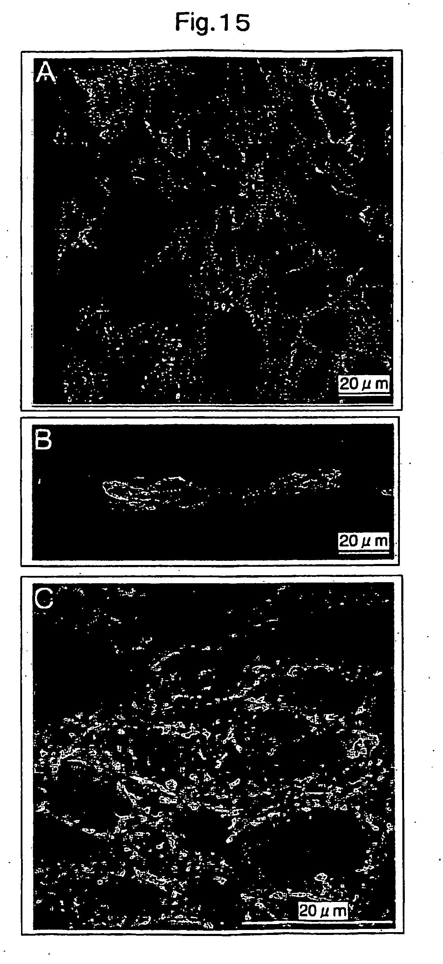

Fig. 15 shows histological evidence for establishment of satisfactory electrical connection between two myocardial cell sheets in vitro.
   Laser confocal microscopy of overlaid MCSs (day 3) after immunohistological stain. The MCSs were triple-stained with antiactinin antibody (green), anticonnexin 43 antibody (red), and TOTO-3 to stain the nucleus. (A) Top view. (B) Side view. (C) Top view observed at high magnification. Note that the myocardial cells formed a confluent sheet and that connexin 43 was clearly observed at the cell junctions.

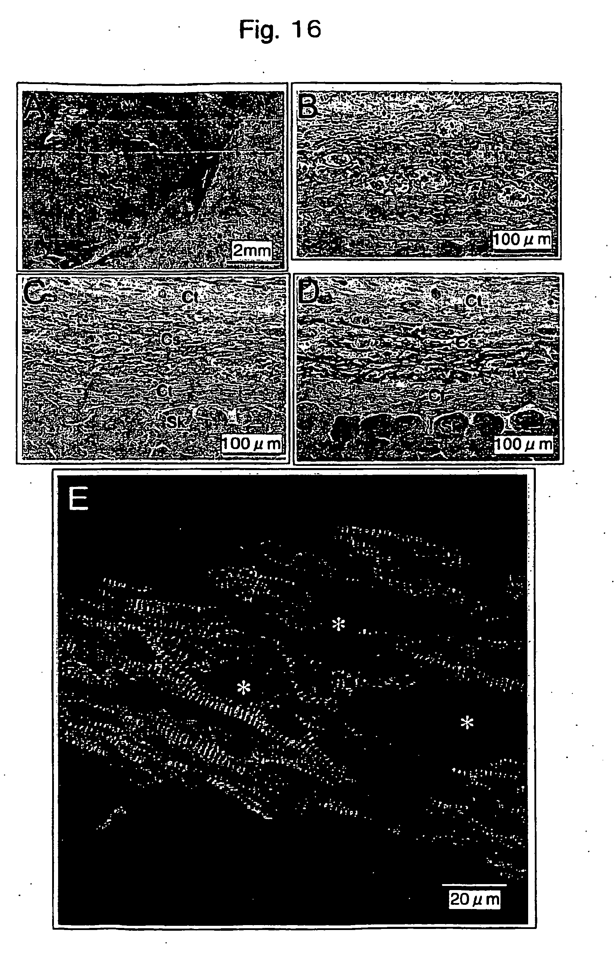

Fig. 16 shows the transplantation of three overlaid myocardial cell sheets in vivo.
   The three overlaid MCSs were transplanted into a nude rat on the subcutaneous tissue and the samples were observed on day 14. (A) The transplanted area (black dotted line) showed rhythmical spontaneous beating (200 bpm). (B) H&E staining of the cross-sectional view of the tri-layered myocardial cell sheet graft. Sk, skeletal muscle; Ct, connective tissue; Cs, transplanted three overlaid MCSs. (C) Azan staining of serial sections of one sample. (D) A cross-sectional view at high magnification. Note that microvessels are apparent in the transplanted MCSs. *Microvessels. (E) Triple staining of the transplanted three overlaid MCSs, as described in connection with Fig. 13. Note that the transplanted myocardial cells show a well-organized sarcomere with a coincident direction of orientation.

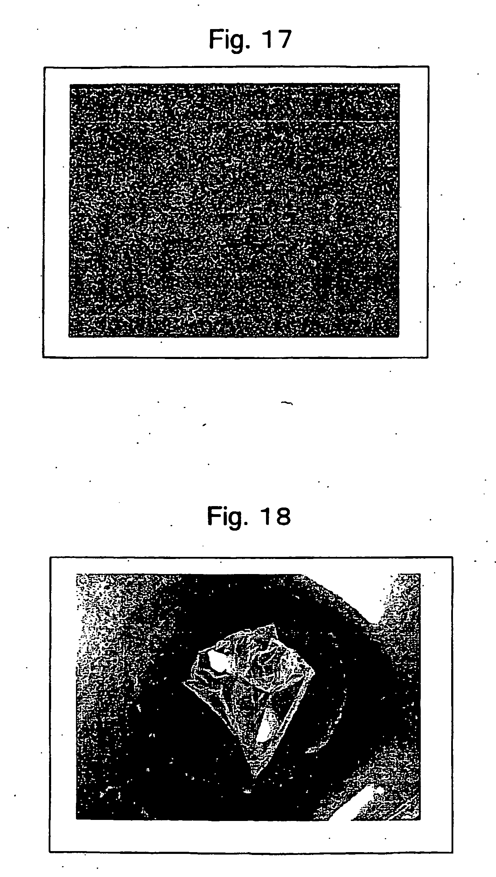

Fig. 17 shows the result of optical microscopy (x100 magnification) of rabbit corneal epithelial cells cultured on a fibrin sheet.
Fig. 18 shows a cultured rabbit corneal epithelial cell sheet as detached with a scraper.

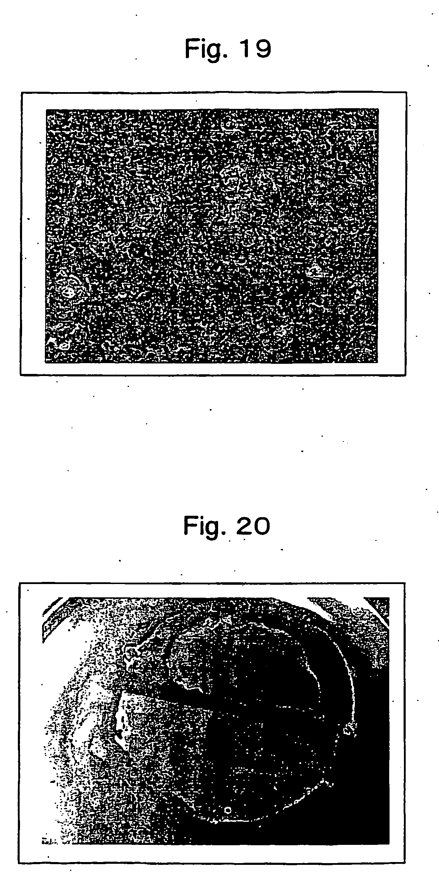

Fig. 19 shows the result of optical microscopy (x100 magnification) of rabbit oral mucosal epithelial cells cultured on a fibrin sheet.
Fig. 20 shows a cultured rabbit oral mucosal epithelial cell sheet as detached with a scraper.

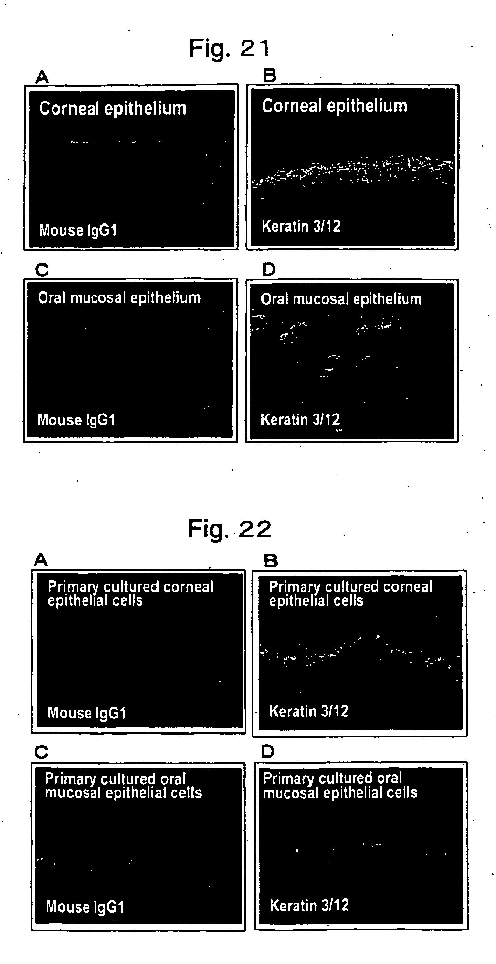

Fig. 21 shows the result of staining keratin 3/12 in rabbit cornea and oral mucosal epithelium as positive controls.
   (A) A nuclear staining image of corneal epithelium.
   (B) A staining image of nuclei and keratin 3/12 of corneal epithelium.
   (C) A nuclear staining image of oral mucosa.
   (D) A staining image of nuclei and keratin 3/12 of oral mucosa.
Fig. 22 shows the immunostaining of cell sheets prepared using rabbit corneal epithelial cells and oral mucosal epithelial cells.
   (A) A nuclear staining image of a corneal epithelial cell sheet.
   (B) A staining image of nuclei and keratin 3/12 of a corneal epithelial cell sheet.
   (C) A nuclear staining image of an oral mucosal epithelial cell sheet.
   (D) A staining image of nuclei and keratin 3/12 of an oral mucosal epithelial cell sheet.

In the cultured epithelium sheets, the corneal epithelium was overlaid and stained at the areas where keratin 3/12 was localized. The oral mucosal epithelium was also overlaid and the areas where keratin 3/12 was localized were weakly stained. The rabbit corneal epithelium and oral mucosal epithelium cultured on fibrin sheets were overlaid and they expressed keratin. Accordingly, it was demonstrated that cultured epithelium having properties similar to those of normal tissues can be prepared using these sheets.

### Best Mode for Carrying Out the Invention

The present invention provides a composition for use in coating with fibrin a surface of a substrate for cell sheet preparation, the composition comprising fibrinogen and thrombin.

Fibrin is a poorly soluble fraction produced by specific hydrolysis of the A alpha-chain and B beta-chain of fibrinogen by thrombin to release fibrinopeptides A and B. The reactive residues on fibrin which participate in aggregation of fibrin monomers are hydrogen-bonded to one another to form a fibrin polymer. The fibrin polymer can gel with a certain configuration.

Fibrinogen is a glycoprotein having a molecular weight of about 340, 000, and is composed of paired sets of three types of subunits: A alpha-chain, B beta-chain and gamma-chain having molecular weights of 65,000 ± 1,000, 55,000 and 47,000, respectively, which are bonded to one another through S-S bonding. The Arg-Gly bonding in fibrinogen is hydrolyzed by thrombin to release fibrinopeptides A and B from the A alpha-chain and the B beta-chain, respectively, whereby fibrinogen is converted into fibrin.

Thrombin is a protease which can act on fibrinogen to produce fibrin. In the composition of the present invention, thrombin may be present in a catalytically effective amount for converting fibrinogen into fibrin.

Fibrinogen and thrombin are preferably derived from human in view of the fact that a cell sheet prepared by cultivating cells on a fibrin coating formed with them is intended to be used in the human body. However, fibrinogen and thrombin are not to be limited to the human origin and may be derived from other animals such as monkey, pig, mouse, rat, baboon, canine, feline, sheep or bovine which are already on the market.

Fibrinogen and thrombin to be used in the invention may be commercially available products. For example, Tissiel Kit from Baxter can be used. Tissiel Kit contains human fibrinogen, human thrombin, calcium chloride dihydrate and aprotinin.

Preferably, the composition of the present invention further contains calcium chloride (which may be in the form of a hydrate), aprotinin, physiological saline and the like.

An example of the composition of the present invention (per 16 ml) is as follows:

| | |
|---|---|
| fibrinogen | 45 to 180 mg; |
| thrombin | 0.2 to 0.8 U; |
| calcium chloride dihydrate | 0.5 to 1.0 mg; |
| aprotinin | 1500 to 6000 U; and |
| physiological saline | 16 ml. |

In the composition, it is recommended to store fibrinogen and fibrin in separate containers and to mix them immediately prior to use, because the formation of fibrin begins to occur upon the reaction of thrombin with fibrinogen. To fibrinogen may be added serum albumin, amino acetic acid, aprotinin, tyloxapol, sodium chloride and sodium citrate. To thrombin may be added serum albumin, amino acetic acid and sodium chloride. It is also recommended to store calcium chloride dihydrate in a container separate from the container for fibrinogen and to mix them immediately prior to use, because ionized calcium accelerates the hydrolysis of fibrinogen. For example, calcium chloride dihydrate may be dissolved in a solution for use in the dissolution of thrombin (hereinafter, referred to as "a dissolution solution for thrombin") immediately prior to use and be stored in a container. Aprotinin may be previously added to fibrinogen or, alternatively, may be dissolved in a solution for use in the dissolution of fibrinogen (hereinafter, referred to as "a dissolution solution for fibrinogen") immediately prior to use and be stored in a container, because aprotinin can inhibit the polymerization of fibrinogen. Here, an example of the method of using of the composition will be described. First, fibrinogen is dissolved in a dissolution solution for fibrinogen containing aprotinin to prepare solution A. Then, thrombin is dissolved in a dissolution solution for thrombin containing calcium chloride dihydrate to prepare solution B. Solutions A and B and physiological saline are mixed together and the mixed solution is applied onto a surface of a substrate for cell sheet preparation.

The composition of the present invention can be used to coat a surface of a substrate for cell sheet preparation with fibrin.

Accordingly, the present invention provides a substrate for cell sheet preparation, a surface of the substrate being coated with fibrin.

The substrate may be of any type, as long as cells can be cultured on it. Examples of the substrate include a culture dish, a Petri dish, a culture plate having 6 to 96 wells, and Celldex LF (SUMILON). The material for the substrate may be exemplified by, but is not limited to, glass, modified glass, polystyrene, polymethyl methacrylate, and ceramics.

For manufacture of the substrate for use in the cell sheet preparation, the composition of the present invention may be applied onto a surface of the substrate to form a fibrin coating thereon. An example of the procedure will be described in detail below.

Fibrinogen, thrombin, calcium chloride dihydrate, aprotinin and physiological saline are mixed together and the mixed solution is then applied onto a surface of the substrate. The substrate is then allowed to stand for an appropriate time period (usually 1 to 3 hours) at room temperature to form fibrin thereon. The resulting substrate may be stored under sterile conditions at 4°C until it is used as a substrate for cell sheet preparation.

The present invention provides a method for manufacturing a cell sheet, comprising culturing cells on a fibrin-coated surface of a substrate until the cells reach confluency; continuing the cultivation of the cells for a sufficient time period to cause the degradation of fibrin at the bottom of the cells; and detaching the cultured cells from the substrate surface in a sheet-like form to give a cell sheet. A film-like sheet can be obtained by raking the cells which have been grown on a culture dish densely until they reach confluency. As used herein, the term "confluency" means the state where cells are placed densely without leaving any gap and it can be observed under a microscope.

Examples of the cell to be cultured include, but are not limited to, myocardial cell, skeletal myoblast, mature skeletal muscle cell, smooth muscle cell, bone marrow stromal cell, corneal epithelial cell, oral mucosal epithelial cell and dermal cell. For the cultivation of the cells on a culture dish to confluency, there are two approaches: one approach is by spreading cells of a single type; and the other approach is by spreading multiple types of cells simultaneously. For the cultivation of a single type of cells to confluency, there are two approaches: one approach is to plate a small amount of monoclonal cells having proliferation potency on a culture dish and then grow the cells until they reach confluency; the other approach is to plate a large amount of polyclonal cells having poor proliferation potency on a culture dish and, when they adhere onto the bottom of the culture dish, grow the cells until they reach confluency. As one example of the former approach, cells of an immortalized cell line (e.g., C2C12 strain cells derived from murine skeletal myoblasts, CMG cells, etc.) are plated in a small amount and grown on a culture plate until the cells reach confluency. As one example of the latter approach, myocardial cells, skeletal myoblasts, bone marrow stromal cells and the like are harvested from cardiac muscle, skeletal muscle, smooth muscle, bone marrow and the like, respectively, by primary culture techniques, the cells are selectively collected by means of a cell sorter, percoll or adhesion-based separation technique to increase the cell purity, and then a sufficient amount of the cells are plated on a culture dish. As one example of the approach for growing multiple types of cells to confluency, fibroblasts are mixed to myocardial or skeletal muscle cells before a cell sheet is formed from them. In this case, even if the number of the myocardial or skeletal muscle cells used is insufficient, fibroblasts which have high proliferation potency invade into the gaps among the myocardial or skeletal muscle cells and the entire bottom surface of the culture dish is covered with either type of cells, thus achieving a confluent state. In this manner, even cells which are difficult to harvest in a sufficient amount and which have poor proliferation potency can be grown to permit easy formation of a cell sheet by co-cultivation of "bridge-cells" such as fibroblasts. As such bridge-cells, not only fibroblasts but also smooth muscle cells and endothelial cells may be used. Depending on the type of cells used as "bridge", the strength and stretching property of a cell sheet can be modified for intended use.

The cells may be derived from human and non-human animals (e.g., monkey, pig, mouse, rat, baboon, canine, feline, sheep or bovine). The cells may be harvested directly from the source such as an animal or they may be cultured cells of an established or unestablished cell line.

The manufacture of a cell sheet can be achieved by culturing cells on the fibrin-coated surface of a substrate until the cells reach confluency; continuing the cultivation of the cells for a sufficient time period to cause the degradation of fibrin at the bottom of the cells; and detaching the cultured cells from the substrate surface in a sheet-like form to give a cell sheet. The cultivation of the cells may be conducted by any method or under any condition as long as the cultivation is conducted on the fibrin-coated surface of a substrate. The cultivation may be continued until the cells reach confluency and the fibrin is degraded to the extent that the cells can be detached from the substrate surface in a sheet-like form. If it is required to culture the cells for a prolonged period of time before sheet formation, an appropriate amount of aprotinin may be added to the culture several days before sheet manipulation. In this manner, the cultivation can be prolonged by a desired number of days without causing degradation of fibrin. Generally, the cells are cultured in a culture medium until they become confluent, and the cultivation is continued for an additional three to four days in a culture medium without aprotinin. During the cultivation, degradation of the fibrin at the bottom of the cells occurs spontaneously due to the cultured cells. In the cultivation, a substance capable of degrading fibrin (e.g., plasmin) may be added to the culture medium to intentionally control the degradation rate of fibrin. Thereafter, the culture medium may be aspirated off and the resulting cell sheet may be detached from the substrate in a film-like form using detaching means such as a scraper. After the cell sheet is detached, a few drops of a fresh culture medium may be applied onto the cell sheet to unfold or smooth out the sheet.

The cultured cells detached in a sheet-like form may be overlaid to form a multiple layers. An example of the procedure for overlaying the cell sheets is described below.

From a first cell sheet which has been unfolded on a culture dish, the culture medium is further aspirated off, and the cell sheet is allowed to stand in a saturated steam incubator at 37°C for an appropriate time (e.g., 15-30 min.). During this time period, the first cell sheet adheres to the culture dish. A second cell sheet as just detached from a culture dish is aspirated along with the culture medium by means of a pipette and then applied onto the first cell sheet fixed on the culture dish. A few drops of the culture medium are gently applied onto the second cell sheet placed in a shrunken state on the unfolded first cell sheet, whereby the second cell sheet can be unfolded while being overlaid on the first cell sheet. The same procedure is repeated to overlay one cell sheet on another.

By using the method of the present invention to form a cell sheet of various cell types or to overlay cell sheets, tissue grafts for a variety of organs can be generated in vitro. Use of the tissue grafts thus prepared enables the establishment of analytical procedures in vitro at the cellular to tissual level.

The cell sheets manufactured by the method of the present invention can be used in the field of regenerative medicine or in biological activity study on an agent.

As the cell sheets for use in regenerative medicine, there may be mentioned a myocardial cell sheet, a corneal epithelial cell sheet, an oral mucosal epithelial cell sheet, a dermal cell sheet and the like. A myocardial cell sheet can be used for treatment of heart failure and arrhythmia resulting from cardiac infarction and various types of myocarditis and cardiomyopathy and as a material for cardiac muscle transplantation. A corneal epithelial cell sheet and an oral mucosal epithelial cell sheet can be used as materials for keratoplasty. A dermal cell sheet can be used for the treatment of wounds resulting from burns and injuries and the like. It may also be possible to use a fibroblast cell sheet in therapy for wound cure promotion.

The biological activity test of an agent may be exemplified by pharmacological activity test, toxicity test and biding activity test of an agent. Examples of the binding activity of an agent include ligand-receptor binding activity and antibody-antigen binding activity. In comparison with the conventional methods for examining the change in cell behavior that results from addition of various agents to a culture medium for cell cultivation, the addition of such various agents to a cell sheet culture medium to examine the effect on the cell sheet enables examining not only the effect on cells themselves but also the effect on intercellular structure and construction. It is also possible to examine such effects of an agent at the cellular level, as well as at the organ level. Cell sheets derived from different human organs can be transplanted onto organs of immunodeficient animals (e. g. , nude mice, skid mice, nude rats) and, after administration of an agent to the transplantation model animals, the state of the cell sheets can be examined to predict the effect of the agent on human organs in vivo.

By the biological activity tests of agents using the cell sheet manufactured by the method of the present invention, candidate substances for medicines and agricultural chemicals having desired biological activities can be screened.

The present invention will be described in great detail with reference to the following examples. However, it should be understood that these examples are for illustrative purposes only and that the scope of the invention is not limited thereto.

The commercial suppliers and preparation methods of the materials used in the examples are as follows:
Culture dishes (FALCON 35 3001, diameter = 3.5 cm);
Wistar rats (Japan CLEA);
2.5% Trypsin (GIBCO 15090-046);
Collagenase (SIGMA C-5138);
DNase I (SIGMA DN-25);
FBS (JRH Bioscience);
Penicillin, streptomycin, amphotericin B (GIBCO 15240-062);
Medium 199 (ICN Biomedicals 1023126);
DMEM (GIBCO 12100-046);
C2C12 derived from murine skeletal myoblasts (purchased from ATCC);
CMG cells (a cell line produced by cloning an immortalized line of murine bone marrow cells that acquired the ability to be transformed to cardiac cells by treatment with 5-azacytidine in Cardiopulmonary Division, Department of Internal Medicine, Keio University School of Medicine);
Nude rats (Japan CLEA);
Rabbit anti-mouse connexin antibody (SIGMA C6219);
Mouse anti-actinin antibody (SIGMA A7811);
TRITC-conjugated pig anti-rabbit IgG antibody (DAKO R 0156);
Alexa488-conjugated goat mouse IgG antibody (Molecular Probes A-11029);
Rabbits (Japanese white rabbits, female, about 3 kg, Shiraishi Laboraty Animal Care Company (Shiraishi Jikken-doubutsu Shiikujo));
SHEM
   ·DMEM/F12: Gibco BRL D-MEM/F12 (1 pack/for 1L, 12400-016; 15.6 g/unit; containing HEPES);
   ·NaHCO₃: Waco (concentration in use: 2.5 g/l);
   ·Insulin: SIGMA human recombinant expressed in E. coli; I-0259; 50 mg/unit (concentration in use: 5 µg/ml));
   ·Human-EGF: Gibco BRL Recombinant Human EGF; 13247-010; 100 µg/unit (concentration in use: 10 µg/ml);
   ·Cholera toxin: SIGMA c-2012; 1 mg/unit (concentration in use: 1 µg/ml);
   ·0.5% DMSO: SIGMA DIMETHYL SULFOXIPE; D-2650; 5 ml x 5 tubes/unit);
   ·15% FCS: Sanko Junyaku (Vitromex); VMS 1500; Lot, F000210802; 500 ml DMEM (Gibco);
Fetal bovine serum (Nichirei);
3T3 cells (American Type Culture Collection);
Aprotinin (Wako);
Gentamicin, penicillin, anphotericin B (Wako);
Dispase II (Godo Shusei);
DMEM/F12 (Gibco);
Trypsin (Gibco);
EDTA (Gibco);
Normal Donkey Serum (CHEMICON; S30-100ML; Lot, 23031387; 100 ml);
BSA (Sigma);

DAPI (Sigma).

### [Example 1] Coating of culture dish with human fibrin

Human fibrinogen (90 mg), thrombin (0.4 U), calcium chloride dihydrate (0.59 mg) and aprotinin (3000 U) (these components were used as Tissiel 1-ML Kit (Baxter)) and physiological saline (16 ml) were mixed together and quickly spread onto the bottom of a 35-cm culture dish (0.3 ml/dish). The dish was allowed to stand for 1 hour at room temperature while it was kept level. Thereafter, the culture dish on which fibrin had formed and the Tissiel dilution solution had solidified at the bottom was stored at 4°C while it was kept sterile. Stored in this state, the culture dish is effective for use for about 2 months. Forty to fifty 3. 5-cm culture dishes could be prepared using the Tissiel 1-ML Kit.

### [Example 2] Cell culture on fibrin-coated culture dishes

### (1) Rat myocardial cells

The ventricles were removed from 1 day-old neonatal Wister rats and enzymatically treated with 0. 03% trypsin, 0.03% collagenase and 20 µg/ml DNase I to isolate ventricular myocytes. Two milliliters of medium 199/DMEM supplemented with 10% FBS and penicillin (50 U/ml) /streptomycin (50 µg/ml) /amphotericin B (25µg/ml) and the cells (2 x 10⁶ cells) were injected into each of the fibrin-coated 3.5-cm culture dishes, and the cells were cultured in a 5% CO₂ incubator at 37°C.

### (2) C2C12 cells derived from murine skeletal myoblasts

Cell line C2C12 derived from murine skeletal myoblasts purchased from ATCC was cultured using DMEM culture medium supplemented with 10% FBS in a 5% CO₂ incubator at 37°C and passaged at 80% confluency.

### (3) Mature skeletal muscle-like cells derived from murine C2C12 cells

The C2C12 cells (1 x 10⁷ cells) which had been passaged according to the procedure (2) were seeded in a 75-cm flask and DMEM (20 ml) supplemented with 5% horse serum was added. The culture medium was replaced by a fresh one at a frequency of once or twice a week while checking the state of the cells.

### (4) Bone marrow stromal cells

The bone marrow was removed under sterile conditions from the thigh bone of mice. The nucleated cells were plated on fibrin-coated 3.5-cm culture dishes at a density of 1 x 10⁷ cells/dish and 3 ml of a mesenchymal stem cell growth medium (PT-3001) (Sanko Junyaku) was added. The medium was replaced by a fresh one at a frequency of once a week.

### [Example 3] Generation of cell sheets from cells

### (1) Preparation of rat myocardial cell sheets

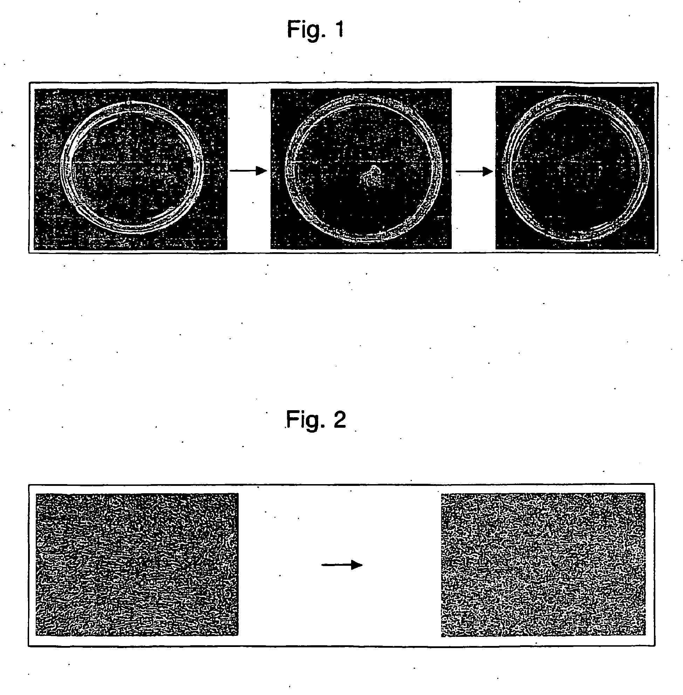

Four days after the primary culture, the cells were beating spontaneously at the bottom of the culture dish in a confluent state. The culture medium was aspirated off and the myocardial cells which had been bonded to one another in a sheet-like form were gently raked with a scraper from the edge toward the center of the culture dish so as not tear the myocardial cell sheet. After the cell sheet was completely detached, a few drops of a fresh culture medium were applied to the shrunken cell sheet carefully to unfold the cell sheet on the culture dish. The generation of the cell sheet from rat myocardial cells is shown in Fig. 1.

### (2) Preparation of cells sheets from C2C12 cells, mature skeletal myoblasts and bone marrow stromal cells

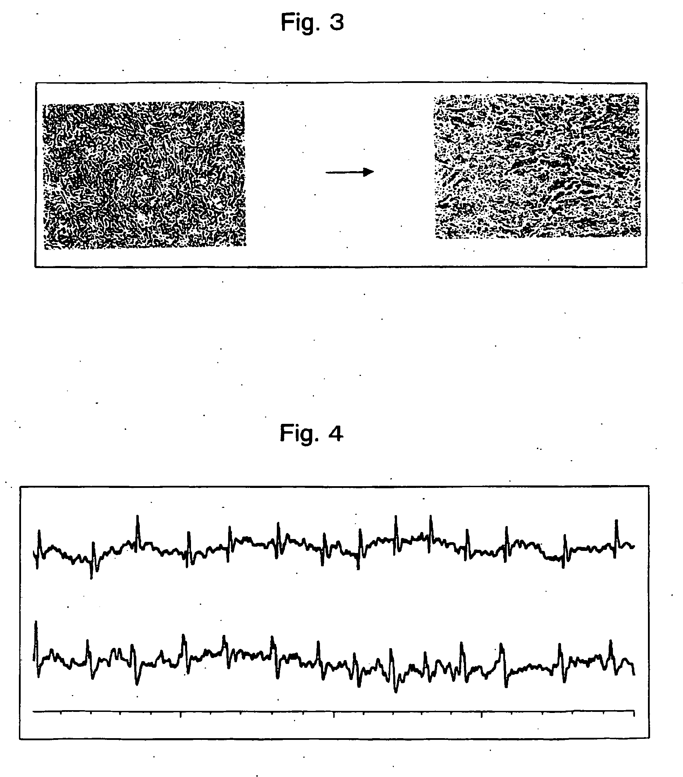

According to the same procedure as in Example 2, cell culture was continued until the cells reached confluency on a fibrin-coated culture dish. After continuing the cultivation for an additional 3 to 4 days, a cell sheet in a film-like form was detached with a scraper in the same manner. The generation process of the cell sheets from C2C12 cells and mature skeletal myoblasts are shown in Figs. 2 and 3, respectively.

### [Example 4] Multiple lamination of cell sheets

A first cell sheet was unfolded on a culture dish by applying a few drops of a culture medium onto the sheet as described in Example 3. The culture medium was aspirated off from the cell sheet as much as possible, and the cell sheet was then allowed to stand in a saturated steam incubator at 37°C for 15 min. During this time period, the first cell sheet adhered to the culture dish with a weak force. Next, a second cell sheet as just detached from a culture dish was aspirated along with a culture medium by means of a 10-ml pipette and applied onto the first cell sheet which had been fixed on the culture dish. A few drops of a fresh culture medium were carefully applied onto the second cell sheet placed in a shrunken state on the unfolded first cell sheet, whereby the second cell sheet was unfolded as it was overlaid on the first cell sheet. The same procedure was repeated to overlay multiple cell sheets successively.

### [Example 5] Functional analysis of myocardial cell sheets

### (1) Electrical activities in vitro

To two partially overlaid myocardial cell sheets was added 1 ml of a culture medium. Culture of the cell sheets was continued while the culture medium was replaced daily with a fresh one. After two days, the two cell sheets were observed under a microscope and they exhibited spontaneous contraction at the same beating rate. The electrocardiogram measured at the both edges of each myocardial cell sheet revealed that the two cell sheets were contracted in the same rhythm (Fig. 4).

### (2) In vivo take

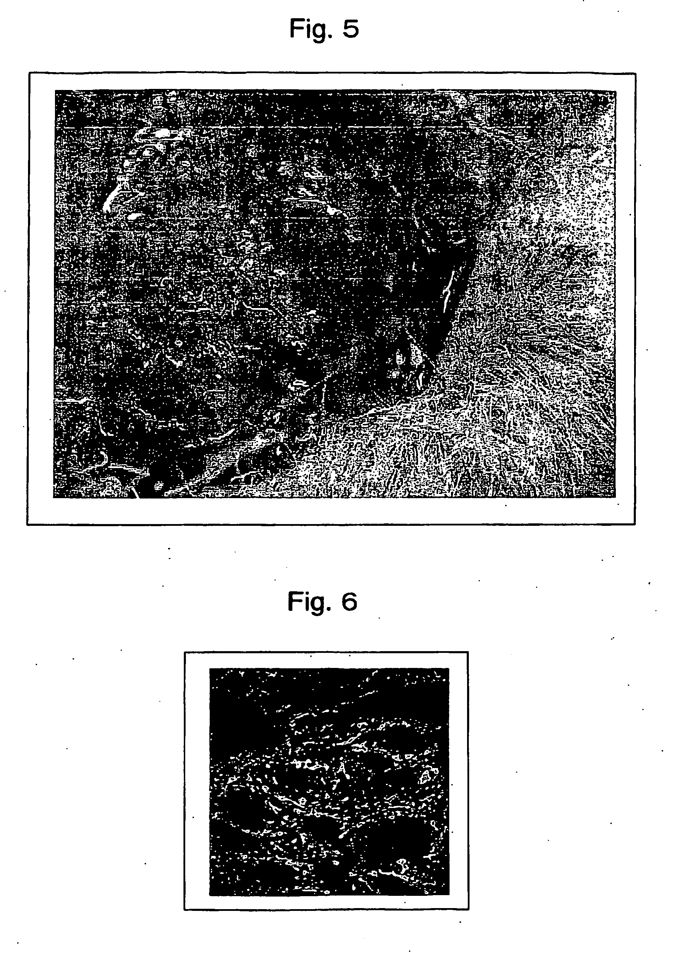

Two overlaid rat myocardial cell sheets were transplanted into 5 week-old nude rats on the subcutaneous tissue. One week after the transplantation, the skin was incised and the myocardial cell sheets were observed (Fig. 5). It was confirmed with the naked eye that the cell sheets showed rhythmical contraction.

### (3) Study of muscle contractile protein and gap junction by immunohistological staining

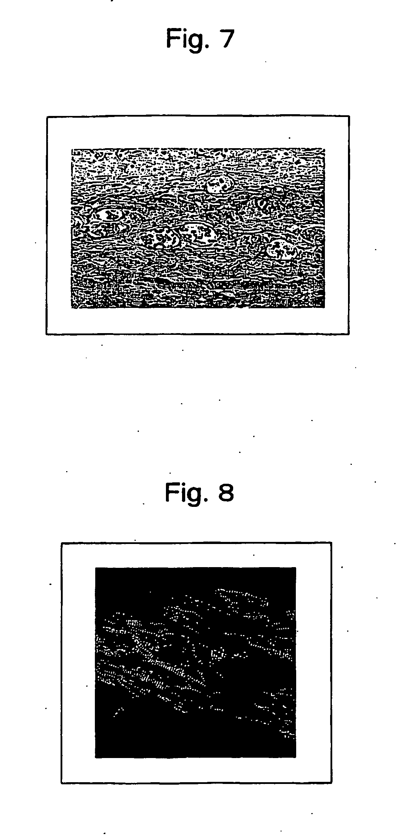

Two days after in vitro preparation of a rat myocardial cell sheet, actinin (a representative contractile protein found in myocardial cells) and connexin 43 (a constitutive protein of gap junction) were immunostained. The result is shown in Fig. 6. Two overlaid rat myocardial cell sheets were transplanted into a 3 week-old nude rat on the subcutaneous tissue in vivo. Seven days later, the cell sheets were removed and subjected to HE staining and immunostaining of actinin and connexin 43. The results are shown in Figs. 7 and 8, respectively. It was demonstrated that the expression of actinin and connexin 43 was maintained satisfactorily both in vivo and in vitro. In the in vivo models, fiber orientation in myocardial cells was more marked than the in vitro models. From the HE staining, it was observed that microvessels invaded into the gaps between the transplanted myocardial cell sheets to supply bloodstream.

### [Example 6] Preparation, transplantation, histological analysis and electrical activity analysis of myocardial cell sheet

### Materials and method

### · Preparation of myocardial cell sheet

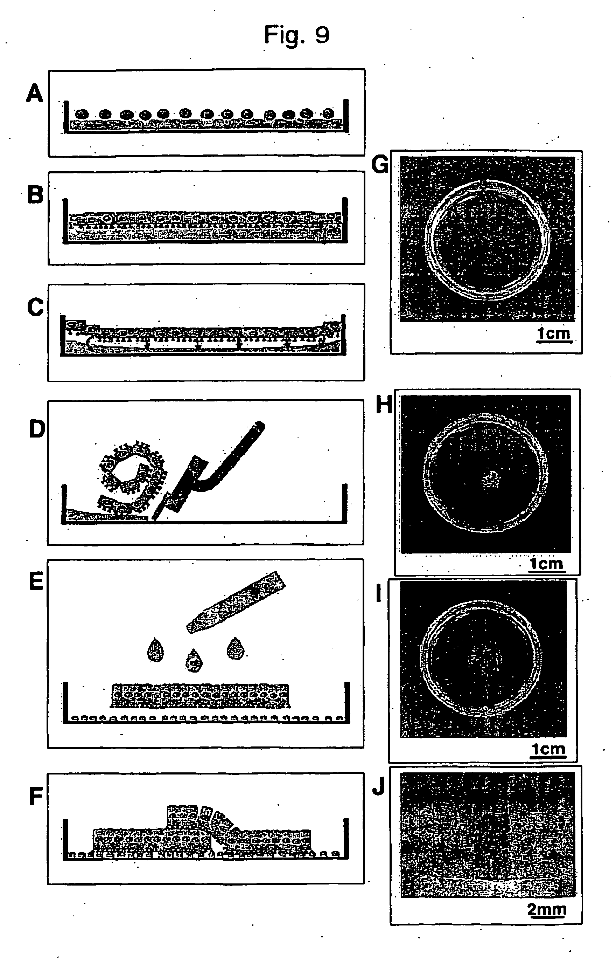

Tissiel (including human fibrinogen, thrombin, calcium chloride and aprotinin) was purchased from Baxter. Human fibrinogen (90 mg), human albumin (20 mg), thrombin (0.4 U), calcium chloride dihydrate (0.59 mg) and aprotinin (3000 U) were diluted with physiological saline (15 ml), and a portion (0.3 ml) of the solution was spread onto a 35-mm culture dish. About 2 hours later, a culture dish of which a surface was coated with fibrin polymer was obtained. This culture dish can be stored under sterile conditions at 4°C for about one month. According to the same procedure described above, myocardial cells could be prepared from ventricular muscle of 1 day-old neonatal Wister rats (Kodama H., Fukuda K., Pan J. et al., Leukemia inhibitory factor, a potent cardiac hypertrophic cytokine, activates the JAK/STAT pathway in rat cardiomyocytes. Circ Res. 1997;81:656-663). The obtained myocardial cells were plated on a fibrin-coated culture dish at a density of 2.8 x 10⁵ cells/cm² (Fig. 9A, B, G). The polymerized fibrin was gradually degraded by non-specific proteases secreted from the cultured cells. In three to seven days after the cultivation was started, the contact between the cells and the surface of the culture dish gradually became sparse (Fig. 9C).

Thus, on day 4, the myocardial cells could be detached from the surface of the culture dish with a cell scraper to give a myocardial cell sheet (Fig. 9D, H). The shrunken myocardial cell sheet was unfolded by being suspended in a culture medium (Fig. 9E, I), and then trimmed in a square shape. Two myocardial cell sheets were partially overlaid (Figs. 9F, J) for subsequent analytical experiments, and co-culture was continued on a laminin-coated culture dish for 1 to 3 days according to the procedure described previously (Murata M., Fukuda K., Ishida H. et al., Leukemia inhibitory factor, a potent cardiac hypertrophic cytokine, enhances L-type Ca2+ current and [Ca2+] i transient in cardiomyocytes. J Mol Cell Cardiol., 1999;31:237-245).

### · Transplantation of myocardial cell sheet graft onto adult rat subcutaneous tissue

All experimental procedures were approved by the Animal Care and Use Committee of Keio University and conformed to the NIH Guide for the Care and Use of Laboratory Animals. After inhalation of diethyl ether, male F344 nude rats (8-week old, n = 10) were topically injected on the dorsal skin with 1% procaine hydrochloride (5-10 ml) and the dorsal skin was incised. Three overlaid myocardial cell sheets were transplanted onto this area.

### · Histological analysis

Immunohistological staining was performed as described previously (Agbulut O., Menot ML., Li Z. et al., Temporal patterns of bone marrow cell differentiation following transplantation in doxorubicin-induced cardiomyopathy, Cardiovasc Res. 2003;58:451-459) by using anti-fibrin antibody (Monosan, the Netherlands), anti-alpha-actinin antibody (Sigma) and connexin 43 antibody (Sigma). The samples were subjected to secondary staining with either Alexa488-labelled anti-mouse IgG antibody (Molecular Probes) or TRITC-labeled anti-rabbit IgG antibody (Dako). In some experiments, staining with Alexa594-labeled phalloidin (Molecular Probes) was also performed. Nuclei were stained with TOTO-3 (Sigma). The stained samples were observed under a confocal laser microscope (LSM510, Zeiss).

### · Analysis of myocardial cell sheet on electrical activities by optical mapping system.

Two myocardial cell sheets were overlaid by the margin with 2-mm width. Extracellular electrical potentials were measured at both ends of each myocardial cell sheet with a pair of bipolar electrodes.

The optical mapping system was applied by using a membrane voltage responsive dye, di-4-ANEPPS (Molecular Probes), to record two-dimensional action potential propagation and evaluate the action potential propagation between the two overlaid myocardial cell sheets.

Di-4-ANEPPS stock solution (20 mM) was dissolved in DMSO containing 20% pluronic F-127 (P-3000, Molecular Probes), and diluted with the culture medium to a final concentration of 10 *µ*M di-4-ANEPPS. The samples were allowed to stand in an incubator at 37°C for 30 min. Thereafter, the culture medium was replaced by Tyrode's solution consisting of (mmol/l) 140 NaCl, 4 KCl, 0.5 MgCl₂, 1.8 CaCl₂, 5 HEPES, 55 D-glucose (pH adjusted to 7.4 with NaOH), and 100 mg/l BSA. The culture dish having the myocardial cell sheets thereon was set in a temperature-controlled perfusion apparatus (37°C) and then placed on the stage of a fluorescence microscope (BX50WI, Olympus, Japan). A high-resolution CCD camera system (MiCAM01, Brain Vision, 192 x 128 points, 3.5 msec time resolution) was used to record signals from the samples at an emission wavelength of 610 nm or longer and an excitation wavelength of 520 nm. The myocardial cell sheet was immobilized using cytochalasin-D (25 µM). Action potentials were recorded under spontaneous beating or pacing stimulation by a bipolar silver chloride electrode. The obtained data was processed with our original analysis program produced using Igor Pro software (Wavemetrics) according to a method described previously (Koura T., Hara M., Takeuchi S. et al., Anisotropic conduction properties in canine atria analyzed by high-resolution optical mapping: preferential direction of conduction block changes from longitudinal to transverse with increasing age. Circulation. 2002;105:2092-2098).

### Results

### · Histological analysis of myocardial cell sheets prepared using fibrin-coated culture dish

For the analysis of myocardial cell sheets prepared by the present method, the inventors prepared cell sheets using a cell scraper at different time points after plating of myocardial cells on fibrin-coated culture dishes (Fig. 10). The myocardial cell sheets could be detached from the culture dishes after three days onward. The detached myocardial cell sheets decreased in diameter by 38 ± 3.6% (n = 30) as compared with the diameter of the culture dishes. Attempts were also made to culture myocardial cells on non-coated, gelatin-, laminin-, or fibronectin-coated culture dishes to confluency and then detach the produced cell sheets with a cell scraper according to the same procedure. However, in those cases, it was impossible to harvest the cells in a sheet form (data not shown). In the experiment under consideration, the inventors defined the time interval (days) between the primary culture and the myocardial cell sheet manipulation as "PX days", and the time interval (days) between the manipulation of cell sheets and the data recording as "SX days". In a myocardial cell sheet manipulated four days after the primary culture (P4-S0), residual fibrin was observed at the bottom of the sheet. However, in a myocardial cell sheet manipulated six days after the primary culture (P6-S0), no residual fibrin was observed.

In a P4-S1 myocardial cell sheet, residual fibrin was still observed between the myocardial cell sheet and the culture dish. However, in a P4-S3 myocardial cell sheet which was obtained after continuing the cultivation for an additional two days, fibrin completely disappeared. Interestingly, when 600 KIU/ml of aprotinin was added to the culture medium, a considerable amount of fibrin was observed to have remained undigested between the cells sheet and the culture dish (H-K). These experimental results suggest that six days is necessary to completely digest residual fibrin between a myocardial cell sheet and a culture dish.

### · Characteristic properties of myocardial cell sheets prepared using fibrin-coated culture dishes

First, the optimal time interval (days) between the initiation of primary culture and the cell sheet manipulation was determined (Fig. 11). The success rate for obtaining myocardial cell sheets began to increase after three days and peaked on day 4 (success rate = 100%, n = 12 each) (Fig. 11A). The diameter of the obtained myocardial cell sheets gradually increased according to the time interval between the primary culture and the sheet manipulation due to the increase in cell density or the mechanism of cell stretching (Fig. 11B). Next, the percentage of spontaneously beating myocardial cell sheets among the myocardial cell sheets manipulated at different time points after the primary culture was determined following three days of continued cultivation after manipulation the sheets. The time interval (PX days) between the primary culture and the cell sheet manipulation did not affect the percentage of resumption of spontaneous beating of the myocardial cell sheets. However, when the occurrence of spontaneous beating was observed under the conditions where the timing of the manipulation of the myocardial cell sheets was fixed to day 4 after the primary culture (P4) and culture of the cell sheets was continued after the sheet manipulation, the percentage of spontaneous beating began to increase significantly after two days and 100% of the myocardial cell sheets exhibited spontaneous beating on day 6 (S6) (6 days SX; Fig. 11C, D). Further, we examined whether the myocardial cell sheets would respond to pacing stimulation to exhibit rhythmic contraction, and it was found that the percentage of myocardial cell sheets that exhibited rhythmic contraction began to increase on day S2 and reached 100% on day S5 (Fig. 11E). Fig. 11F shows the time course of beat frequency (beating rate) of myocardial cell sheets that exhibited spontaneous contraction after their manipulation. Based on these results, it was decided to use a P4-S3 myocardial cell sheet for the subsequent electrophysiological analysis.

### · Comparison of optical mapping system with the approach of using bipolar electrodes for analysis of action potential propagation

We examined the action potential propagation within a myocardial cell sheet and determined whether action potential would propagate between two myocardial cell sheets via junctions. The data of recording electrocardiogram on myocardial cell sheets as measured by contact with bipolar electrodes was compared with the data of recording action potential as measured by an optical mapping system (Fig. 12). In the electrocardiogram recordings, it was observed that sheets A and B exhibited synchronous spontaneous beating. In the electrocardiogram, the QRS complexes in sheets A and B were completely synchronous, suggesting that the two myocardial cell sheets had established an electrical connection. In the recordings of active potential by the optical mapping system, however, it was unexpectedly observed that the action potential arose from the lower left side of sheet A toward the upper right side and propagated to sheet B via the upper junction and spread through sheet B. This observation demonstrates that the propagation of action potential does not follow a direct route at the junction between the two sheets. It was shown that the conduction velocity for action potential at the area indicated by the arrow in Fig. 12D was satisfactory without causing any delay or disturbance (Fig. 12E). Thus, it was found that the optical mapping system is the sole useful technique for analyzing the action potential propagation within a myocardial cell sheet and the electrical connection between two myocardial cell sheets.

### Constitution process of electrical connection between two myocardial cell sheets

To investigate how electrical connection was constituted between two myocardial cell sheets, the inventors analyzed the propagation of action potential in partially overlaid two myocardial cell sheets by the optical mapping system. In P4-S1 myocardial cell sheets, the action potential did not propagate from one miocardial cell sheet to the other (Fig. 13). Interestingly, in some cases (3/10), partial capture of action potential was observed in sheet B (the arrows in Fig. 13D). Fig. 13F is a contour map showing the propagation of action potential by means of isochronal lines. The conduction velocity of the excitation wave front on each line is shown in Fig. 13G, and it was revealed that propagation of action potential was blocked at site t.

These experimental results suggest that electrical connection is locally established between two P4-S1 myocardial cell sheets but stable electrical connection is yet to be established between the two sheets.

In contrast, in P4-S3 myocardial cell sheets, action potential propagated from sheet A to sheet B without conduction delay (Fig. 14). In all of the test samples, satisfactory electrical connection was established between two myocardial cell sheets (n = 10/10), and no conduction delay was observed in the conduction velocity analysis. These experimental results suggest that a co-culture period three days (S3) or longer is necessary to form stable electrical connection between overlaid myocardial cell sheets.

### · Histological study of attachment between two myocardial cell sheets (in vitro model)

An immunofluorescent staining image of P4-S3 myocardial cell sheet is shown in Fig. 15. Sarcomeres are clearly visible in the myocardial cells. Cx43 was localized at the junctions of the myocardial cells. The sarcomeres seemed to have such a tendency that they were oriented in the coincident direction upon mutual contact of myocardial cells. The two overlaid myocardial cell sheets were approximately 15 ± 2 µm thick. The two cell sheets were completely connected and it was impossible to recognize their boundary.

### · In vivo transplantation model and histological study of three overlaid myocardial cell sheets

Three overlaid myocardial cell sheets were transplanted together into nude rats on the subcutaneous tissue and observed after 14 days. The cell sheet showed strong periodic contraction (Fig. 16). The HE- and Azan-staining revealed that the transplanted myocardial cell sheets were sandwiched between host-derived connective tissues (Fig. 16B, C). The myocardial cell sheets were 102 ± 11 µm thick, thicker than when the in vitro cultivation of three overlaid myocardial cell sheets was continued. Confocal laser microscopy showed that the size of each myocardial cell in vivo was greater than that of the myocardial cells in myocardial cell sheets prepared in vitro. In addition, rich neovascularization was observed in the transplanted myocardial cell sheets (Fig. 16D). This vasculature had diameters in the range of 10 to 25 µm, which is not at the capillary level but at the microvessel level. The sarcomere in the myocardial cells was well organized and the distribution of myocardial cells had such a tendency that they were oriented in the coincident direction (Fig. 16E). These findings indicate that myocardial cell sheets obtained using fibrin-coated culture dishes remained functional in the tissues, had tissue structures similar to those of the normal heart and had good contraction potency.

### Discussion

Since methods for preparing cell sheets using PIAAm-coated responsive culture dishes were reported, cell sheet engineering has been advanced as one of the approaches of two- or three-dimensional tissue engineering in a variety of organs. As a consequence, cell sheet engineering is beginning to assume a more dominant position in the field of regenerative medicine now. Hitherto, preparation of cell sheets from vascular endothelial cells, hepatocytes, renal endothelial cells, corneal epithelial cells and the like using temperature-responsive culture dishes has been reported (Shimizu T., Yamato M., Kikuchi A. et al., Cell sheet engineering for myocardial tissue reconstruction. Biomaterials. 2003;24:2309-2316).

The method reported herein by the present inventors has several advantageous characteristics. First, cell sheets can be prepared easily merely by using readily available fibrin without the need of specialized facilities. Accordingly, the method enables cell sheets of optimal size and various shapes to be prepared without the need of specialized techniques. Second, cell sheets can be prepared from any type of adhesive cells. This is because that even cells that find difficulty adhering to conventional uncoated cell culture dishes or fibronectin-coated culture dishes exhibit extremely good adhesive properties onto fibrin-coated culture dishes. Third, cell sheets can be harvested rapidly. At the time when the present method was developed, there was a fear that the cellular structure of myocardial cell sheets might be impaired during the detachment of the cells in a sheet-like form with a cell scraper. However, there was no visible cell necrosis under microscopic observation. The reasons for this result are assumed as follows: the attachment of myocardial cells to the culture dishes is gradually loosened as fibrin is steadily degraded by non-specific proteases secreted from the myocardial cells; and the trace amount of residual fibrin may serve a cushion-like function to protect the cells against physical damage during harvest. The present inventors have confirmed that the residual fibrin completely disappears within a total of seven days after the primary culture of the myocardial cells However, the length of this period may vary depending on the nature of proteases secreted from the cells themselves, cell density at the point of time when the cells reach confluency, and the like.

As an exemplary enzyme that is secreted from the liver in vivo and which has potent proteolytic effect, plasmin is well known (Ritchie DG, Levy BA, Adams MA et al. Regulation of fibrinogen synthesis by plasmin-derived fragments of fibrinogen and fibrin: an indirect feedback pathway. Proc Natl Acad Sci USA 1982;79:1530-1534). Even if fibrin remains in myocardial cell sheets at the point in time when the cell sheets are detached from the culture dishes, the residual fibrin is believed to disappear by the action of endogenous plasmin after transplantation. Based on the results shown herein, the method for preparing various types of cell sheets using fibrin-coated culture dishes is considered as a practical and simple procedure in myocardial cell sheet engineering.

In order that myocardial cell sheets can be used as transplantation grafts useful in the field of myocardial tissue engineering, good propagation and communication of action potential within or between the myocardial cell sheets are required. For this purpose, the optical mapping system is believed to be an extremely effective means for detailed examination of action potential propagation. In two overlaid myocardial cell sheets which had been confirmed to exhibit completely synchronized contraction under microscopic observation, analysis by the optical mapping system revealed that active potential propagated in such a way as to go around the junctions via areas where good electrical connection was established. As a consequence, electrophysiological studies by the optical mapping system are considered to be extremely effective for analyzing the presence of electrical connection between a host and a graft after the transplantation of the graft onto the heart of the host. According to the experiments shown herein, it suggested that three days is necessary to establish satisfactory electrical connection between two myocardial cell sheets. Experiments for studying the process of electrical connection establishment in myocardial cell sheet-transplanted models using the optical mapping system are also underway.

In conclusion, it is considered that the method for preparing myocardial cell sheets and the method for electrophysiological analysis of myocardial cell sheets, as disclosed herein, will largely contribute to advances in the field of regenerative medicine for heart and other organs.

### [Example 6] Generation of cell sheets from cells

### (1) Preparation of corneal epithelial cell sheet

The following procedure was taken to prepare a corneal epithelial cell sheet on a fibrin sheet that had been applied to a surface of a tissue culture dish (IWAKI).
1) The Descemet's membrane and iris were scraped from a rabbit with a swab and the cornea was removed therefrom.
2) The resulting sample was split into 12 pieces and placed on the fibrin-coated inner well with the epithelium side down.
3) SHEM (0.6 ml) supplemented with aprotinin (666 KIU/ml) was added to the inner well. When the epithelium began to grown, the amount of addition of SHEM (supplemented with 666 KIU/ml of aprotinin) was changed to 1 ml
4) DMEM + FCS (+), a culture medium used for cultivation of 3T3 cells, was replaced by 2 ml of SHEM (supplemented with 666 KIU/ml of aprotinin).
5) When the epithelium reached confluency (which usually requires 1 to 2 weeks), air lift was performed (for about 1 week). The culture medium used is SHEM without aprotinin.
6) The cultured epithelium was detached with a scraper.

A photographic image (x100) of the rabbit corneal epithelial cells grown on the fibrin sheet observed under an optical microscope is shown in Fig. 17. A photographic image of the cultured epithelium sheet detached with a scraper is shown in Fig. 18. Using rabbit corneal epithelial cells cultured on a fibrin sheet, a thick epithelium sheet could be prepared by means of co-cultivation of 3T3 cells and air lifting.

### (2) Preparation of oral mucosal epithelial cell sheet

The following procedure was taken to prepare an oral mucosal epithelial cell sheet on a fibrin sheet that had been applied to a tissue culture dish (IWAKI).
1) The oral mucosa was collected from a rabbit (2 mm x 2mm x 1 mm specimens from two sites).
2) The tissue was washed (three times with a medium supplemented with gentamicin and antibiotics/antifungal agents at RT, each lasting for 15 min, then once with antibiotics-loaded PBS(-)at RT for 15 min).
3) The sample was treated with Dispase II (at 37°C for 1 hour).
4) The epithelium was removed from the parenchyma and placed in DMEM/F12 + FCS (-).
5) The sample was treated with trypsin-EDTA (at RT for 8 min).
6) The sample was centrifuged at 1500 rpm for 5 min [then plated on an inner well at a density of 2 x 10⁵ cells/ml SHEM (supplemented with 666 KIU/ml of aprotinin)].
7) DMEM + FCS (+), a culture medium used for cultivation of 3T3 cells, was replaced by 2 ml of SHEM (supplemented with 666 KIU/ml of aprotinin).
8) When the epithelium reached confluency (which usually takes 1 to 2 weeks), air lift was.performed (for about 1 week). The culture medium used was SHEM without aprotinin.
9) The cultured epithelium was detached with a scraper.

A photographic image (x100) of the rabbit oral mucosal epithelial cells grown on the fibrin sheet observed under an optical microscope is shown in Fig. 19. A photographic image of the cultured epithelium sheet detached with a scraper is shown in Fig. 20. Using rabbit oral mucosal epithelial cells cultured on a fibrin sheet, a thick epithelium sheet could be prepared by means of co-cultivation of 3T3 cells and air lifting.

### (3) Amex embedding fixation of fibrin sheet culture

The epithelium sheet was embedded by the Amex method according to the following procedure.
1) The epithelium sheet was placed in a net.
2) The sample sheet was immersed in acetone at 4°C
3) The sheet was transferred along with acetone to -20°C and fixed for 24 hours.
4) The acetone was replaced by a fresh aliquot (4°C) to impregnate the sheet for 15 min.
5) The acetone was replaced by a fresh aliquot (room temperature) to impregnate the sheet for 15 min.
6) The acetone was replaced by methyl benzoate to impregnate the sheet for 15 min. This step was repeated twice.
7) The methyl benzoate was replaced by xylene to impregnate the sheet for 15 min. This step was repeated twice.
8) The sheet was impregnated with paraffin for 1 hour. This step was repeated three times.
9) The sheet was embedded in paraffin.

### (4) Immunostaining of fibrin sheet culture

With respect to each of the samples of rabbit corneal tissue, oral mucosal tissue, cultured corneal epithelium and cultured oral mucosal epithelium, a paraffin-embedded section was immunostained according to the following procedure.
1) The section was treated with 3% H₂O₂ at room temperature for 30 min.
2) Blocking (10% normal donkey serum - 1% BSA - 0.01M PBS) of the section was performed at room temperature for 1 hour.
3) The section was reacted with a primary antibody (AE5: PROGEN 61807: 1/1000). Isotype control (mouse IgG1: Dako: 1/500) O/N.
4) The section was reacted with a secondary antibody (donkey anti-Ms IgG-FITC, Jackson: 711-095-152: 1/50) at room temperature for 30 min.
5) Nuclear staining (DOJINDO: 1 µg/ml DAPI) was performed at room temperature for 5 min.

The immunostaining (AE5) of the rabbit corneal epithelial tissue and oral mucosal epithelial tissue is shown in Fig. 21. Blue: nuclear staining (DAPI). Green: AE5 staining (keratin 3/12). The keratin 3/12 in the epithelium of the cornea and the oral mucosa as positive controls showed staining of epithelial cells in both tissues (Fig. 21B and D).

The immunostaining (AE5) of the rabbit cultured corneal epithelial cells and cultured oral mucosal epithelial cells is shown in Fig. 22. In the cultured two types of epithelium, the corneal epithelium formed multiple layers and the staining of keratin 3/12 was observed (Fig. 22B and D). The oral mucosal epithelium also formed multiple layers, but the staining of keratin 3/12 was observed weakly.

As mentioned above, rabbit corneal epithelium and oral mucosal epithelium cultured on fibrin sheets formed multiple layers and expressed keratin. Accordingly, it was demonstrated that cultured epithelium having properties similar to those of normal tissue could be prepared.

### Industrial Applicability

Using the cell sheets manufactured by the method of the present invention, it becomes possible to create analysis models for various conditions (e.g., arrhythmia in myocardial cells) and to transplant tissues at the clinical level, for example, in regenerative medicine. In addition, the cell sheets manufactured by the method of the present invention can be used to conduct various biological activity tests for agents, thus enabling screening for candidate substances as medicines and agricultural chemicals having desired biological activities.

## Claims

1. A composition for use in coating with fibrin a surface of a substrate for cell sheet preparation, the composition comprising fibrinogen, thrombin and physiological saline, wherein the content of fibrinogen is 45-180 mg per 16 ml of physiological saline and the content of thrombin is 0.2-0.8 U per 16 ml of physiological saline.

2. A method for manufacturing a substrate having a surface coated with fibrin for cell sheet preparation, comprising coating the surface of the substrate with the composition according to claim 1.

3. A method for manufacturing a cell sheet which contains substantially no fibrin, comprising culturing cells on a fibrin-coated surface of a substrate until the cells reach confluency; continuing the cultivation of the cells for a sufficient time period to cause the degradation of fibrin at the bottom of the cells; and detaching the cultured cells from the substrate surface in a sheet-like form to give a cell sheet.

4. The method according to claim 3, further comprising overlaying the detached sheet-like cultured cells.

5. The method according to claim 3 or 4, wherein the cell sheet is to be used in the field of regenerative medicine or in a biological activity test of an agent.

6. The method according to claim 3, in which the surface of the substrate is coated with fibrin using the composition according to claim 1.

7. The method according to claim 6, further comprising overlaying the detached sheet-like cultured cells.

8. The method according to claim 6 or 7, wherein the cell sheet is to be used in the field of regenerative medicine or in a biological activity test of an agent.

## Patentansprüche

1. Zusammensetzung zur Verwendung in Beschichtung einer Oberfläche eines Trägers zur Herstellung einer Zellage mit Fibrin, die Zusammensetzung umfasst Fibrinogen, Thrombin und physiologische Kochsalzlösung, wobei der Gehalt an Fibrinogen 45-180 mg pro 16 ml physiologische Kochsalzlösung und der Gehalt an Thrombin 0,2-0,8 U pro 16 ml physiologische Kochsalzlösung ist.

2. Verfahren zur Herstellung eines Trägers, der eine mit Fibrin beschichtete Oberfläche für die Herstellung einer Zellage aufweist, umfassend Beschichtung der Oberfläche des Trägers mit der Zusammensetzung nach Anspruch 1.

3. Verfahren zur Herstellung einer Zellage, die im Wesentlichen kein Fibrin enthält, umfassend das Kultivieren von Zellen auf einer Fibrin beschichteten Oberfläche eines Trägers bis die Zellen Konfluenz erreichen; Beibehalten der Kultivierung der Zellen für eine ausreichende Zeitspanne bis ein Abbau von Fibrin am unteren Ende der Zellen bewirkt ist; und Ablösen der kultivierten Zellen von der Trägeroberfläche in einer schichtähnlichen Form um eine Zellage zu erhalten.

4. Verfahren nach Anspruch 3, weiterhin umfassend das Übereinanderlegen der abgelösten schichtähnlichen kultivierten Zellen.

5. Verfahren nach Anspruch 3 oder 4, wobei die Zellage in dem Bereich der regenerativen Medizin oder in einem biologischen Aktivitätstest für einen Wirkstoff genutzt werden soll.

6. Verfahren nach Anspruch 3, in dem die Oberfläche des Trägers unter Verwendung der Zusammensetzung nach Anspruch 1 mit Fibrin beschichtet ist.

7. Verfahren nach Anspruch 6, weiterhin umfassend das Übereinanderlegen der abgelösten schichtähnlichen kultivierten Zellen.

8. Verfahren nach Anspruch 6 oder 7, wobei die Zellage in dem Feld der regenerativen Medizin oder in einem biologischen Aktivitätstest für einen Wirkstoff genutzt werden soll.

## Revendications

1. Composition pour une utilisation dans le revêtement avec une fibrine d'une surface d'un substrat pour la préparation d'une feuille cellulaire, la composition comprenant du fibrinogène, de la thrombine et une solution physiologique saline, dans laquelle la teneur en fibrinogène est de 45 à 180 mg pour 16 ml de solution physiologique saline et la teneur en thrombine est 0,2 à 0,8 U pour 16 ml de solution physiologique saline.

2. Procédé pour fabriquer un substrat ayant une surface revêtue de fibrine pour la préparation d'une feuille cellulaire, comprenant revêtir la surface du substrat de la composition selon la revendication 1.

3. Procédé pour fabriquer une feuille cellulaire qui ne contient sensiblement pas de fibrine, comprenant la mise en culture de cellules sur une surface revêtue de fibrine d'un substrat jusqu'à ce que les cellules atteignent la confluence ; la poursuite de la culture des cellules pendant une période de temps suffisante pour entraîner la dégradation de fibrine au fond des cellules ; et le détachement des cellules mises en culture de la surface du substrat sous une forme analogue à une feuille pour donner une feuille cellulaire.

4. Procédé selon la revendication 3, comprenant en outre la superposition des cellules cultivées, détachées, analogues à une feuille.

5. Procédé selon la revendication 3 ou 4, dans lequel la feuille cellulaire doit être utilisée dans le domaine de la médecine régénérative ou dans un test d'activité biologique d'un agent.

6. Procédé selon la revendication 3, dans lequel la surface du substrat est revêtue de fibrine en utilisant la composition selon la revendication 1.

7. Procédé selon la revendication 6, comprenant en outre la superposition des cellules cultivées, détachées, analogues à une feuille.

8. Procédé selon la revendication 6 ou 7, dans lequel la feuille cellulaire doit être utilisée dans le domaine de la médecine régénérative ou dans un test d'activité biologique d'un agent.
